# EUROPÄISCHE PATENTSCHRIFT

(11) **EP 0 854 142 B1**
(45) Veröffentlichungstag und Bekanntmachung des Hinweises auf die Patenterteilung: **18.09.2002**
(21) Anmeldenummer: 98100111.8
(22) Anmeldetag: 07.01.1998
(51) Int. Cl.: C07D 231/14

(54) **Verfahren zur Herstellung von 1-Alkyl-pyrazol-5-carbonsäureestern**
Process for the preparation of 1-alkylpyrazole-5-carboxylic esters
Procédé de préparation d'esters de l'acide 1-alkylpyrazole-5-carboxylique

(30) Priorität: 16.01.1997 DE 19701277
(43) Veröffentlichungstag der Anmeldung: 22.07.1998
(73) Patentinhaber: BAYER AG, 51368 Leverkusen (DE)
(72) Erfinder: Müller, Nikolaus, Dr., 40789 Monheim (DE); Heuer, Lutz, Dr., 41542 Dormagen (DE); Kanellakopoulos, Johannes, Dr., 41542 Dormagen (DE); Sattler, Andreas, Dr., 40229 Düsseldorf (DE); Heise, Klaus-Peter, Dr., 51519 Odenthal (DE)

(56) Entgegenhaltungen:
- EP-A- 0 029 363
- EP-A- 0 029 364
- DD-A- 264 210
- PATENT ABSTRACTS OF JAPAN vol. 10, no. 199 (C-359) [2255] , 11.Juli 1986 & JP 61 040266 A (MORISHITA SEIYAKU K.K.), 26.Februar 1986,
- W. THEILHEIMER: "Synthetic Methods of Organic Chemistry" 1962 , S. KARGER XP002058626 * vol. 16, 428; page 204 *
- W. THEILHEIMER: "Synthetic Methods of Organic Chemistry" 1984 , S. KARGER XP002058627 * vol. 38, 412; page 166 *
- W. THEILHEIMER: "Synthetic Methods of Organic Chemistry" 1975 , S. KARGER XP002058628 * vol. 2, 368; page 128 *
- W. THEILHEIMER: "Synthetic Methods of Organic Chemistry" 1952 , S. KARGER XP002058629 * vol. 6, 403; page 148 *
- PATENT ABSTRACTS OF JAPAN vol. 9, no. 59 (C-270) [1782] , 15.März 1985 & JP 59 196868 A (UBE KOSAN K.K.), 8.November 1984,
- A.R. KATRITSKY ET AL.: "comprehensive heterocyclic chemistry" 1984 , PERGAMON PRESS XP002058630 * vol. 5, pages 277-282 *
- PATENT ABSTRACTS OF JAPAN vol. 095, no. 008, 29.September 1995 & JP 07 118238 A (UBE IND., LTD.), 9.Mai 1995,

## Beschreibung

Die vorliegende Erfindung betrifft ein Verfahren zur Herstellung von 1-Alkyl-, insbesondere auch 1,3-Dialkyl-pyrazol-5-carbonsäureestern, aus dem Enolat von 2,4-Dicarbonsäureestern und N-Alkylhydraziniumsalzen.

Es ist bereits bekannt, 1-Alkyl-pyrazol-5-carbonsäureester durch Alkylierung von Pyrazol-3-carbonsäureestem mit Alkylierungsmitteln (z.B. Alkylhalogeniden, Dialkylsulfaten oder Alkyltosylaten) herzustellen.

So beschreiben EP-OS 463 756 und EP-OS 526 004 die Herstellung von 1-Methyl-3-n-propyl-pyrazol-5-carbonsäureethylester durch die Umsetzung von 3-n-Propylpyrazol-5-carbonsäureethylester mit Dimethylsulfat. Offenbar entsteht hier ein Gemisch aus den beiden isomeren N-Methyl-pyrazolen, das aufwendig mittels chromatographischer Methoden aufgetrennt werden muß.

In der DE-OS 19 27 429 wird die Herstellung von 1-Methyl- und 1-Ethyl-3-propylpyrazol-5-carbonsäureethylester aus 3-Propyl-pyrazol-5-carbonsäureethylester durch Alkylierung mit Dimethylsulfat oder Triethyloxoniumtetrafluoroborat beschrieben. Es finden sich keine Angaben zum eventuellen Auftreten von Isomeren, ihrer Trennung oder der Gesamtausbeute des gewünschten Produktes, obwohl aufgrund der bis dahin bekannten Literatur immer mit der Entstehung von Isomerengemischen gerechnet werden muß. So führt die Methylierung von 3,4-Dimethyl-pyrazol-5-carbonsäuremethylester mit Methyliodid und Natriummethylat als Hilfsbase zu einem Gemisch der isomeren Trimethylpyrazolcarbonsäureester, die man erst "nach mehrfacher Rektifikation" in die Isomeren trennen konnte (J. Prakt. Chem. 126, 198 (1930)). Die Ethylierung von 3-Methylpyrazol-5-carbonsäure-ethylester mit Ethylbromid/Natrium in absolutem Alkohol ergibt ein Gemisch aus 1-Ethyl-3-methyl-pyrazol-5-carbonsäureester und 1-Ethyl-5-methyl-pyrazol-3-carbonsäureethylester im Verhältnis von ca. 1:3. D.h. das häufig gewünschte 1,3-Dialkylisomere wird im deutlichen Unterschuß gebildet und erst nach dreimaliger Destillation rein erhalten (Chem. Ber. 59, 603 (1926)).

Die andere, weithin verbreitete Methode, 1-Alkyl-pyrazol-5-carbonsäureester herzustellen besteht in der Umsetzung von 2,4-Diketocarbonsäureestern mit N-Alkylhydrazinen. Auch hier werden Isomerengemische erhalten, die in der Regel überwiegend das häufig unerwünschte Isomere enthalten, was dann ebenfalls ein aufwendiges Trennverfahren nötig macht. So ergibt die Umsetzung von 2,4-Dioxopentancarbonsäureethylester mit Methylhydrazin ein 1:1-Gemisch aus 1,5-Dimethylpyrazol-3-carbonsäureester und dem entsprechenden 2,5-Dimethylisomeren (Austr. J. Chem. 36, 135-147 (1983)). Andere Autoren berichten für diese Umsetzung noch ungünstigere Verhältnisse von 35:65 (Chem. Ber. 59, 1282 (1926)), die in vergleichenden Laborversuchen bestätigt wurden. Die gleichen Autoren haben mit analogen veretherten Enolen, z.B. mit O-Ethylacetonoxalester und Methylhydrazin, noch schlechtere Ergebnisse erzielt (Isomerenverhältnis 15:85). In der Regel werden bei diesen Umsetzungen entweder die freien Hydrazine mit dem Diketoester oder das Natriumsalz des Diketoesters (Enolat) und Hydrazinsalze eingesetzt, aus denen das Hydrazin mit Laugen wie Natriumhydroxid oder Soda in Freiheit gesetzt wird.

JP 0711838 beschreibt ein Verfahren zur selektiven Herstellung von 1-Alkyl-pyrazol 5-carbonsäureestern mittels Temperaturkontrolle.

Die bei Herstellungsverfahren des Standes der Technik anfallenden Reaktionsgemische können nicht destillativ aufgearbeitet werden, weil sie Nebenprodukte enthalten, die eine destillative Trennung der beiden gebildeten Isomeren unmöglich machen.

Es besteht deshalb immer noch das Bedürfnis nach einem Verfahren zur selektiven Herstellung möglichst isomerenfreier 1-Alkyl-pyrazol-5-carbonsäureester.

Es wurde nun ein Verfahren zur Herstellung von 1-Alkyl-pyrazol-5-carbonsäureestern der Formel (I) gefunden 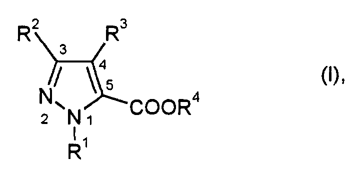 in der
- R¹ und R⁴: unabhängig voneinander jeweils für geradkettiges oder verzweigtes C₁-C₆-Alkyl, C₃-C₇-Cycloalkyl oder gegebenenfalls substituiertes C₇-C₁₂--Aralkyl und
- R² und R³: unabhängig voneinander jeweils für Wasserstoff, geradkettiges oder verzweigtes C₁-C₆-Alkyl, C₃-C₇-Cycloalkyl oder gegebenenfalls substituiertes C₇-C₁₂-Aralkyl stehen,
das dadurch gekennzeichnet ist, daß man das Enolat eines 2,4-Diketocarbonsäureesters der Formel 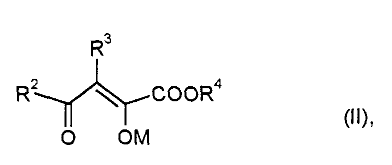 in der
- R², R³ und R⁴: die bei Formel (I) angegebene Bedeutung haben und
- M: für ein Äquivalent eines Metallatoms steht,
in Gegenwart eines Lösungsmittels zu einem N-Alkylhydraziniumsalz der Formel (III) gibt

R¹-NH₂-NH₂^{⊕} R⁵COO^{θ} (III),

in der
- R¹: die bei Formel (I) angegebene Bedeutung hat und
- R⁵: zusammen mit dem COO^{θ}-Teil für das Anion einer organischen Säure steht.

C₇-C₁₂-Aralkyl und das daraus bevorzugte Benzyl sowie C₆-C₁₀-Aryl (später genannt) und das daraus bevorzugte Phenyl (später genannt) können beispielsweise jeweils bis zu zwei Substituenten aus der Gruppe der Halogenatome und der C₁-C₄-Alkylreste enthalten.

Als Diketocarbonsäureesterenolate der Formel (II) sind solche bevorzugt, bei denen die Reste R² und R³ unabhängig voneinander jeweils für Wasserstoff, geradkettiges oder verzweigtes C₁-C₄-Alkyl, C₃-C₆-Cycloalkyl oder gegebenenfalls substituiertes Benzyl stehen sowie solche, bei denen der Rest R⁴ für geradkettiges oder verzweigtes C₁-C₄-Alkyl steht.

Besonders bevorzugt sind 2,4-Diketocarbonsäureesterenolate der Formel (II), bei denen R² und R⁴ jeweils für C₁-C₄-Alkyl und R³ für H stehen.

M in der Formel (II) steht bevorzugt für ein einwertiges Metallatom oder ein Äquivalent eines zweiwertigen Metallatoms. Beispiele sind Lithium, Natrium, Kalium, Calcium und Magnesium. Besonders bevorzugt sind Natrium, Lithium und Magnesium.

Von den N-Alkylhydraziniumsalzen der Formel (III) sind solche bevorzugt, bei denen R¹ für geradkettiges oder verzweigtes C₁-C₄-Alkyl oder gegebenenfalls substituiertes Benzyl und R⁵ für geradkettiges oder verzweigtes C₁-C₆-Alkyl, C₃-C₇-Cycloalkyl, C₇-C₁₂-Aralkyl oder C₆-C₁₀-Aryl stehen, die gegebenenfalls wie oben angegeben substituiert sein können.

R⁵ kann zusammen mit dem COO^{θ}-Teil auch für ein Anion einer mehrbasischen organischen Säure stehen. Beispiele sind Anionen von Oxal-, Malon-, Bernstein-, Glutar-, Adipin-, Malein-, Fumar-, Äpfel-, Wein- und Zitronensäure, wobei es sich jeweils um Mono- oder Polyanionen handeln kann. Solche Anionen können einen oder mehrere COO^{θ}-Teile und gegebenenfalls zusätzlich auch COOH-Reste enthalten. Im Falle von Anionen von mehrbasischen organischen Säuren kann das Alkylhydrazin im Verhältnis zur Säure in äquimolaren Mengen oder in Mengen, die der Anzahl der Säuregruppen entspricht, eingesetzt werden.

Die Diketoesterenolate der Formel (II) können z.B. aus den entsprechenden, reinen, isolierten 2,4-Diketoestern durch Zugabe eines Alkoholats hergestellt werden. Als Alkoholate kommen z.B. solche in Frage, die der Formel (IV) entsprechen

M(OR⁶)ₙ (IV),

in der
- M: die bei Formel (II) angegebene Bedeutung hat,
- R⁶: für C₁-C₄-Alkyl steht und
- n: der Wertigkeit von M entspricht.

Die Herstellung der Diketoesterenolate der Formel (II) kann in einem Lösungsmittel, beispielsweise in Wasser und/oder einem C₁-C₄-Alkohol erfolgen.

Diketoesterenolate der Formel (II) können auch nach der üblichen Methode durch Kondensation eines Methylalkylketons der Formel (V) 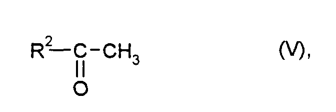 in der
- R²: die bei Formel (I) angegebene Bedeutung hat,
mit einem Oxalester der Formel

R⁴OOC-COOR⁴ (VI)

in der
- R⁴: die bei Formel (I) angegebene Bedeutung hat,
hergestellt werden. Auch hier arbeitet man in Gegenwart eines Alkoholats, beispielsweise eines Alkoholats der Formel (IV) und eines Lösungsmittels. Die dabei erhaltene rohe Reaktionsmischung kann direkt in die Umsetzung mit dem N-Alkylhydraziniumsalz der Formel (III) eingesetzt werden.

Als Lösungsmittel für die Umsetzung der Methylalkylketone der Formel (V) mit Oxalestern der Formel (VI) und Weiterreaktion mit den N-Alkylhydraziniumsalzen der Formel (III) kommen beispielsweise Alkohole wie Methanol, Ethanol, Isopropanol, n-Propanol und n-, i-, s- und t-Butanol in Frage. Das Alkoholat der Formel (IV) kann durch Auflösen eines Alkali- oder Erdalkalimetalls M in dem Alkohol hergestellt werden, der dem Alkoholat der Formel (IV) entspricht.

Das Hydraziniumsalz der Formel (III) kann z.B. durch Mischen eines Alkylhydrazins der Formel (VII)

R¹-NH-NH₂ (VII),

in der
- R¹: die bei Formel (I) angegebene Bedeutung hat,
mit einer Carbonsäure der Formel (VIII) hergestellt werden

R⁵-COOH (VIII),

in der
- R⁵: die bei Formel (III) angegebene Bedeutung hat.

Man kann dabei gegebenenfalls unter Zusatz eines Lösungsmittels, z.B. eines Alkohols arbeiten.

Zur Herstellung von Verbindungen der Formel (I) werden das Enolat der Formel (II) und das N-Alkylhydraziniumsalz der Formel (III), beide gegebenenfalls in Form der bei ihrer Herstellung anfallenden Lösung, zusammengefügt. Eventuell zur Ausfällung neigende Anteile der Enolate oder der N-Alkylhydraziniumsalze können durch Erwärmen und/oder Zugabe von weiterem Lösungsmittel wieder in Lösung gebracht oder gehalten werden. Die alkoholische Enolatlösung wird zur vorgelegten N-Alkylhydraziniumsalzlösung hin zugegeben.

Die insgesamt eingesetzten Lösungsmittelmengen werden im allgemeinen so gewählt, daß rührbare Suspensionen oder Lösungen vorliegen. Die Lösungsmittelmenge pro Mol Reaktionsansatz kann z.B. zwischen 100 und 2000 ml liegen. Bevorzugt beträgt diese Menge 200 bis 1000 ml, besonders bevorzugt 250 bis 500 ml.

Das Molverhältnis für die Umsetzung der Diketoesterenolate der Formel (II) mit den N-Alkylhydraziniumsalzen der Formel (III) kann in weiten Grenzen schwanken, beispielsweise von 5:1 bis 1:5. In einer bevorzugten Ausführungsform werden etwa äquimolare Mengen Diketoesterenolat der Formel (II) und N-Alkylhydraziniumsalz der Formel (III) zur Reaktion gebracht, beispielsweise 0,9 bis 1,1 Mole Diketoesterenolat der Formel (II) pro Mol N-Alkylhydraziniumsalz der Formel (III).

Die Alkoholatmenge zur Herstellung des Diketoesterenolats kann ebenfalls in weiten Grenzen schwanken. Vorzugsweise ist sie zumindest äquimolar.

Wird der Diketoester in einem vorgeschalteten Schritt wie oben beschrieben aus einem Keton der Formel (V) und einem Oxalsäureester der Formel (VI) hergestellt, so können auch deren Molverhältnisse variieren. Bevorzugt arbeitet man mit einem leichten Unterschuß, z.B. je mit einem Unterschuß von 1 bis 10 Mol-% an dem Keton der Formel (V) bezogen auf den Oxalester der Formel (VI) und das Alkoholat der Formel (IV). Die beiden letzteren werden bevorzugt in etwa äquimolaren Mengen zueinander eingesetzt. Die Molverhältnisse betragen dann beispielsweise 0,9 bis 0,99 Mol Keton der Formel (V) zu 0,9 bis 1,1 Mol Oxalsäureester der Formel (VI) zu 1 Mol Alkoholat der Formel (IV).

Bei der Herstellung des N-Alkylhydraziniumsalzes der Formel (III) aus einem Alkylhydrazin der Formel (VII) und einer Carbonsäure der Formel (VIII) kann das Molverhältnis der beiden Einsatzstoffe ebenfalls in weiten Grenzen schwanken. Bevorzugt ist eine Menge von 1 bis 1,5 Mol Säure pro Mol Alkylhydrazin. Besonders bevorzugt ist der Einsatz äquimolarer Mengen Alkylhydrazin und Carbonsäure.

Ein Überschuß an Carbonsäure der Formel (VIII) ist vorteilhaft, wenn Alkoholat der Formel (IV) im Überschuß vorliegt. Dann kann dieser Alkoholatüberschuß mit dem Säureüberschuß neutralisiert werden.

Die Reaktionstemperaturen für die Umsetzung der Reaktionspartner der Formel (II) und (III) können z.B. zwischen -20 und +100°C liegen, bevorzugt zwischen 0 und 80°C, besonders bevorzugt zwischen 20 und 50°C. Dies gilt auch für die Temperaturen während der Nachrührzeiten.

Die Reaktionszeiten (Zusammengeben der Reaktionspartner + Nachrührzeit) können z.B. zwischen 0,5 und 12 Stunden, bevorzugt zwischen 1 und 8 Stunden, besonders bevorzugt zwischen 2 und 5 Stunden betragen. Für das erfindungsgemäße Verfahren werden bevorzugt folgende Verbindungen der Formel (II) eingesetzt: 2,4-Diketopentancarbonsäureethylester als Natrium-, Lithium-, Kalium- oder Magnesiumenolat, 2,4-Diketohexancarbonsäureethylester, 2,4-Diketoheptancarbonsäureethylester, 2,4-Diketooctancarbonsäureethylester und 2,4-Diketo-3-ethylpentancarbonsäureethylester (jeweils in Form ihrer Natrium-, Lithium-, Magnesium- oder Kaliumenolatsalze) und Methyl-, n-Propyl-, i-Propyl- und n-, i-, s- und t-Butylester der zuvor genannten Diketocarbonsäuren ebenfalls in Form der genannten Enolatsalze.

N-Alkylhydraziniumsalze der Formel (III) sind bevorzugt Methylhydraziniumformiat, -acetat, -propionat, -butyrat, -isobutyrat, -benzoat, -oxalat und -succinat, N-Propylhydraziniumforminat, -acetat, -propionat, -butyrat, -isobutyrat und -benzoat, Ethylhydraziniumformiat, -acetat, -propionat, -butyrat, -i-butyrat, -benzoat, -oxalat und -succinat sowie die Hydraziniumsalze von i-Propyl-, n-Butyl-, t-Butyl-, Benzyl- und n-Pentylhydrazin mit den vorgenannten Carbonsäureanionen.

Eine allgemeine Ausführungsform des erfindungsgemäßen Verfahrens, beispielhaft an der Umsetzung von 2,4-Diketoheptancarbonsäureethylester-Natrium-Salz mit Methylhydraziniumformiat erläutert, ist wie folgt:

Aus 2-Pentanon und Oxalester wird mit Natriumethylat als Hilfsbase in Ethanol das Natriumsalz des 2,4-Diketoheptancarbonsäureethylesters analog bekannter Vorschriften hergestellt (siehe z.B. Organikum, 19. Auflg., S. 490 (1993)). Diese Lösung wird, um Ausfallen des Enolates zu verhindern, auf 50°C gehalten und innerhalb von 1 Stunde zu einer zuvor hergestellten Lösung von Methylhydraziniumformiat in Ethanol gegeben (Methylhydrazin in Ethanol vorlegen, Ameisensäure zutropfen). Man rührt eine angemessene Zeit nach, destilliert das überschüssige Ethanol ab und versetzt mit Wasser und Toluol. Die Toluolphase wird abgetrennt, die wäßrige Phase noch 2 mal mit Toluol extrahiert und nach Vereinigung der organischen Phasen diese mit Wasser reextrahiert. Die toluolische Lösung des Rohpyrazols wird durch Destillation des Lösungsmittels eingeengt und der Rückstand im Vakuum fraktioniert destilliert. Die beiden isomeren Pyrazole lassen sich ohne großen Trennaufwand in reiner Form isolieren.

Es ist ausgesprochen überraschend, daß der Einsatz der N-Alkylhydraziniumsalze anstelle freier Hydrazine zur Herstellung von N-Alkylpyrazolen die Regioselektivität umkehrt. Wie im folgenden Formelschema erläutert wird, wird nach dem Stand der Technik beim Einsatz freier Alkylhydrazine (ob als solche eingesetzt oder durch Alkalilaugen aus ihren Salzen freigesetzt ist unerheblich) das unerwünschte Isomere mit dem Alkylrest am von der Carboxylgruppe entfernteren Stickstoff bevorzugt gebildet, während das erfindungsgemäße Verfahren bevorzugt das gewünschte Isomere (I) liefert, bei dem die Alkylgruppe an dem Stickstoff steht, der der Carbonylgruppe im Pyrazol am nächsten steht.

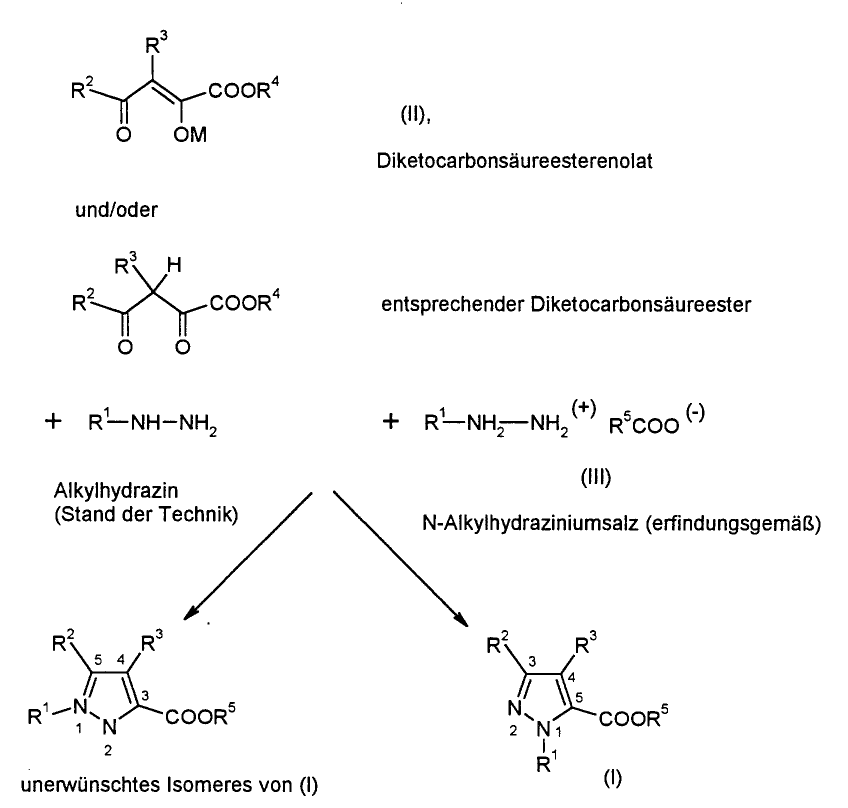

Das erfindungsgemäße Verfahren gestattet die gezieltere Herstellung von substituierten 1-Alkyl-pyrazol-5-carbonsäureestern. Im Vergleich zu der üblichen Methode - Ringschluß von 2,4-Diketoestern mit Hydrazin und anschließende N-Alkylierung - spart dieses Verfahren den Alkylierungsschritt, der zur aufwendigen Zwischenisolierung des Hydrazinumsetzungsproduktes und zur aufwendigen Aufarbeitung des Alkylierungsansatzes zwingt. Das erfindungsgemäße Verfahren ist besonders vorteilhaft durch die Möglichkeit einer einfachen destillativen Trennung zur Isolierung des gewünschten Produktes aus der Reaktionsmischung, vermeidet Abfälle und ist durch seine Kürze energiesparend. Es macht die bekannte Direktumsetzung der Diketoester mit Alkylhydrazinen durch drastisches Verschieben der Isomerenverhältnisse zugunsten des gewünschten Produktes besonders wirtschaftlich.

1-Alkyl-pyrazol-5-carbonsäureester der Formel (I) sind wertvolle Zwischenprodukte zur Herstellung von pharmazeutischen Wirkstoffen mit gefäßverändernder und/oder krampflösender Wirkung (siehe EP-OS 463 756, EP-OS 526 004 und DE-OS 19 27 429) sowie zur Herstellung von Pestiziden mit insektizider und akarizider Wirkung (siehe JP-OS 89-114 466).

### Beispiele

### Beispiel 1

### 1-Methyl-3-n-propyl-pyrazol-5-carbonsäure-ethylester

In einem 1 l-Vierhalskolben wurden 340 g 20 gew.-%ige Natriumethylatlösung in Ethanol vorgelegt und hierzu innerhalb einer Stunde ein Gemisch aus 78 g Pentanon-2 und 146,1 g Oxalsäurediethylester unter Rühren zugetropft. Nach vollständiger Zugabe wurden 100ml Ethanol zugegeben, noch eine Stunde am Rückfluß nachgerührt und dann auf 50°C abgekühlt. Während der Nachrührzeit wurden in einem 11-Vierhalskolben 41,5 g Methylhydrazin und 50 ml Ethanol vorgelegt und hierzu unter Außenkühlung innerhalb von 15 Minuten 41,4 g Ameisensäure getropft. Zu dieser Mischung wurde nun die wie oben angegebene hergestellte rohe 2,4-Diketoheptancarbonsäureethylesterenolat-Lösung gegeben, die während der Zugabe auf 30 bis 40° gehalten wurde, um ein Auskristallisieren des Enolatsalzes zu verhindern. Die Temperatur der Vorlage wurde während der Zugabe zwischen 30 und 35°C gehalten. Die Zugabe war nach 1 Stunde beendet. Die vorliegende Suspension wurde im Laufe der Zugabe der Esterenolat-Lösung immer dünner. Nachdem eine weitere Stunde bei 30°C nachgerührt wurde wurden 4,5 g Ameisensäure zugesetzt, erhitzt und Ethanol bis zu einer Sumpftemperatur von 110°C abdestilliert. Es wurden 505 g Ethanol erhalten. Die gut rührbare Suspension wurde auf Raumtemperatur abgekühlt und mit 400 ml Wasser und 200 ml Toluol versetzt. Die organische Phase wurde abgetrennt und die wäßrige Phase mit 200 ml Toluol extrahiert. Die vereinigten organischen Phasen wurden mit 100 ml Wasser gewaschen und das Toluol abdestilliert. Der Rückstand (173,1 g - Isomerenverhältnis 4,5:1 1-Methyl-3-n-propylpyrazol-5-carbonsäure-ethylester zu 1-Methyl-5-n-propyl-pyrazol-3-carbonsäureethylester), ein tiefbraunes Öl, wurde durch fraktionierte Destillation in die Einzelkomponenten getrennt. Es wurden 98 g des gewünschten Produktes in einer Reinheit von 98,9 Gew.-% (gaschromatographisch bestimmt) erhalten. Das entspricht einer Ausbeute von 56,2 % der Theorie.

### Beispiel 2 (zum Vergleich)

37,3 g 2,4-Diketoheptancarbonsäureethylester wurden in 75 ml Ethanol vorgelegt und innerhalb von 15 Minuten unter Außenkühlung bei 20°C mit einer Lösung von 9,2 g Methylhydrazin in 25 ml Ethanol versetzt. Nach vollständiger Zugabe wurde 1 Stunde am Rückfluß erhitzt und dann wieder auf Raumtemperatur abgekühlt. Die gaschromatographische Untersuchung des Reaktionsgemisches ergab ein Verhältnis von 1-Methyl-5-n-propyl-pyrazol-3-carbonsäureethylester (unerwünschtes Produkt) zu 1-Methyl-3-n-propyl-pyrazol-5-carbonsäureethylester (gewünschtes Produkt) von 4:1. Das Reaktionsgemisch wurde nach diesem Befund nicht mehr aufgearbeitet.

### Beispiel 3

### 1-Methyl-3-n-propyl-pyrazol-5-carbonsäureethylester

In einem 250 ml Vierhalskolben wurden 76,5 g 20 gew.-%ige Natriumethylatlösung vorgelegt und innerhalb von 15 Minuten 41,9 g 2,4-Diketoheptancarbonsäureethylester hinzugegeben. Die so hergestellte Lösung wurde zu einer Mischung aus Methylhydrazin (10,4 g), Essigsäure (13,5 g) und Ethanol (20 ml) innerhalb von 45 Minuten bei 30 bis 40°C Innentemperatur zugegeben. Nach vollständiger Zugabe wurde noch 1 Stunde bei Raumtemperatur nachgerührt und dann das Ethanol abdestilliert Sodann wurden 100ml Toluol und 100ml Wasser zugegeben, die sich bildenden Phasen getrennt und die wäßrige Phase noch zweimal mit je 50 ml Toluol extrahiert. Die vereinigten Toluolextrakte wurden mit 100 ml Wasser reextrahiert, getrocknet und im leichten Vakuum eingeengt. Durch fraktionierte Destillation wurden 31 g isomerenfreier 1-Methyl-3-n-propyl-pyrazol-5-carbonsäureethylester mit einem Siedepunkt von 125 bis 128°C bei 13 mm Druck erhalten. Das entspricht einer Ausbeute von 75 % der Theorie. Das unerwünschte Isomere 1-Methyl-5-n-propylpyrazol-3-carbonsäureethylester wurde als 2. Fraktion in einer Ausbeute von 6,2 g (= 15 % der Theorie) erhalten. Dessen Siedepunkt betrug 166 bis 168°C bei 13 mm Druck.

## Patentansprüche

1. Verfahren zur Herstellung von 1-Alkyl-pyrazol-5-carbonsäureestern der Formel (I) 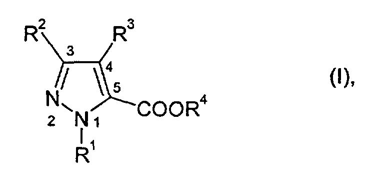 in der
R¹ und R⁴ unabhängig voneinander jeweils für geradkettiges oder verzweigtes C₁-C₆-Alkyl, C₃-C₇-Cycloalkyl oder gegebenenfalls durch bis zu zwei Substitutenten aus der Gruppe der Halogenatome und der C₁-C₄-Alkylreste substituiertes C₇-C₁₂-Aralkyl und
R² und R³ unabhängig voneinander jeweils für Wasserstoff, geradkettiges oder verzweigtes C₁-C₆-Alkyl, C₃-C₇-Cycloalkyl oder gegebenenfalls durch bis zu zwei Substitutenten aus der Gruppe der Halogenatome und der C₁-C₄-Alkylreste substituiertes C₇-C₁₂-Aralkyl stehen,
**dadurch gekennzeichnet, dass** man das Enolat eines 2,4-Diketocarbonsäureesters der Formel 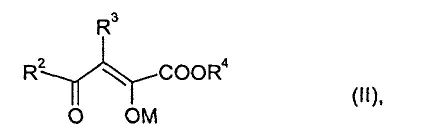 in der
R², R³ und R⁴ die bei Formel (I) angegebene Bedeutung haben und
M für ein Äquivalent eines Metallatoms steht,
in Gegenwart eines Lösungsmittels zu einem N-Alkylhydraziniumsalz der Formel (III) gibt
R¹-NH₂-NH₂^{⊕} R⁵COO^{θ} (III),
in der
R¹ die bei Formel (I) angegebene Bedeutung hat und
R⁵ zusammen mit dem COO^{θ}-Teil für das Anion einer organischen Säure steht.

2. Verfahren nach Anspruch 1, **dadurch gekennzeichnet, dass** es sich bei den Diketocarbonsäureesterenolaten der Formel (II) um solche handelt, bei denen die Reste R² und R³ unabhängig voneinander jeweils für Wasserstoff, geradkettiges oder verzweigtes C₁-C₄-Alkyl, C₃-C₆-Cycloalkyl oder gegebenenfalls substituiertes Benzyl stehen sowie solche, bei denen der Rest R⁴ für geradkettiges oder verzweigtes C₁-C₄-Alkyl steht.

3. Verfahren nach Ansprüchen 1 und 2, **dadurch gekennzeichnet, dass** M in der Formel (II) für Lithium, Natrium, Kalium, Calcium oder Magnesium steht.

4. Verfahren nach Ansprüchen 1 bis 3, **dadurch gekennzeichnet, dass** es sich bei den N-Alkylhydraziniumsalzen der Formel (III) um solche handelt, bei denen R¹ für geradkettiges oder verzweigtes C₁-C₄-Alkyl oder gegebenenfalls substituiertes Benzyl und R⁵ für geradkettiges oder verzweigtes C₁-C₆-Alkyl, C₃-C₇-Cycloalkyl, C₇-C₁₂-Alalkyl oder C₆-C₁₀-Alyl stehen, die gegebenenfalls substituiert sein können.

5. Verfahren nach Ansprüchen 1 bis 4, **dadurch gekennzeichnet, dass** in Formel (III) R⁵ zusammen mit dem COO^{θ}-Teil für ein Anion einer mehrbasischen organischen Säure wie Oxal-, Malon-, Bernstein-, Glutar-, Adipin-, Malein-, Fumar-, Äpfel-, Wein- oder Zitronensäure steht.

6. Verfahren nach Ansprüchen 1 bis 5, **dadurch gekennzeichnet, dass** man die Diketoesterenolate der Formel (II) aus den entsprechenden, reinen, isolierten 2,4-Diketoestern durch Zugabe eines Alkoholats in Gegenwart eines Lösungsmittels herstellt.

7. Verfahren nach Ansprüchen 1 bis 6, **dadurch gekennzeichnet, dass** man zur Ausfällung neigende Anteile der Enolate und/oder der N-Alkylhydraziniumsalze durch Erwärmen und/oder Zugabe von weiterem Lösungsmittel wieder in Lösung bringt oder hält.

8. Verfahren nach Ansprüchen 1 bis 7, **dadurch gekennzeichnet, dass** pro Mol Reaktionsansatz 100 bis 2 000 ml Lösungsmittel vorliegen.

9. Verfahren nach Ansprüchen 1 bis 8, **dadurch gekennzeichnet, dass** das Molverhältnis für die Umsetzung der Diketoesterenolate der Formel (II) mit den N-Alkylhydraziniumsalzen der Formel (III) zwischen 5:1 und 1:5 liegt und das Diketoesterenolat aus einem Methylalkylketon der Formel (V) 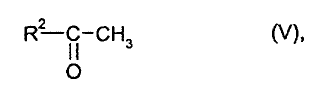 in der
R² die bei Formel (I) angegebene Bedeutung hat,
mit einem Oxalester der Formel
R⁴OOC-COOR⁴ (VI)
in der
R⁴ die bei Formel (I) angegebene Bedeutung hat,
in Gegenwart eines Alkoholats der Formel (IV)
M(OR⁶)ₙ (IV),
in der
M die bei Formel (II) angegebene Bedeutung hat,
R⁶ für C₁-C₄-Alkyl steht und
n der Wertigkeit von M entspricht,
hergestellt wurde, wobei
die Alkoholmenge zumindest äquimolar ist und die Molverhältnisse 0,9 bis 0,99 Mol Keton der Formel (V) zu 0,9 bis 1,1 Mol Oxalsäureester der Formel (VI) zu 1 Mol Alkoholat der Formel (IV) betragen.

10. Verfahren nach Ansprüchen 1 bis 9, **dadurch gekennzeichnet, dass** die Reaktionstemperatur für die Umsetzung der Reaktionspartner der Formeln (II) und (III) zwischen -20 und +100°C und die Reaktionszeiten zwischen 0,5 und 12 Stunden liegen.

## Claims

1. A process for the preparation of 1-alkyl-pyrazole-5-carboxylic acid esters of the formula (I) 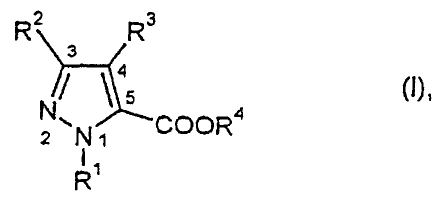 in which
R¹ and R⁴ independently of one another each represent straight-chain or branched C₁-C₆-alkyl, C₃-C₇-cycloalkyl or C₇-C₁₂-aralkyl which is optionally substituted by up to two substituents from the group consisting of halogen atoms and C₁ - C₄- alkyl radicals and
R² and R³ independently of one another each represent hydrogen, straight-chain or branched C₁-C₆-alkyl, C₃-C₇-cycloalkyl or C₇-C₁₂-aralkyl which is optionally substituted by up to two substituents from the group consisting of halogen atoms and C₁ - C₄- alkyl radicals,
**characterized in that** it comprises adding the enolate of a 2,4-diketocarboxylic acid ester of the formula 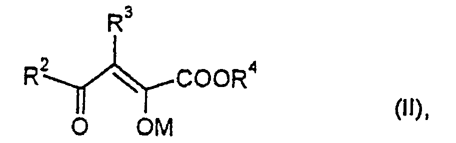 in which
R², R³ and R⁴ have the meaning given in the case of formula (I) and
M represents one equivalent of a metal atom,
to an N-alkylhydrazinium salt of the formula (III)
R¹-NH₂-NH₂^{⊕} R⁵COO^{θ} (III),
in which
R¹ has the meaning given in the case of formula (I) and
R⁵ together with the COO^{θ} moiety, represents the anion of an organic acid, in the presence of a solvent.

2. The process as claimed in claim 1, **characterized in that** the diketocarboxylic acid ester enolate of the formula (II) is one in which the radicals R² and R³ independently of one another in each case represent hydrogen, straight-chain or branched C₁-C₄-alkyl, C₃-C₆-cycloalkyl or optionally substituted benzyl, or one in which the radical R⁴ represents straight-chain or branched C₁-C₄-alkyl.

3. The process as claimed in claims 1 and 2, **characterized in that** M in the formula (II) represents lithium, sodium, potassium, calcium or magnesium.

4. The process as claimed in claims 1 to 3, **characterized in that** the N-alkylhydrazinium salt of the formula (III) is one in which R¹ represents straight-chain or branched C₁-C₄-alkyl or optionally substituted benzyl and R⁵ represents straight-chain or branched C₁-C₆-alkyl, C₃-C₇-cycloalkyl, C₇-C₁₂-aralkyl or C₆-C₁₀-aryl, which can optionally be substituted.

5. The process as claimed in claims 1 to 4, **characterized in that**, in formula (III), R⁵, together with the COO^{θ} moiety, represents an anion of a polybasic organic acid, such as oxalic, malonic, succinic, glutaric, adipic, maleic, fumaric, malic, tartaric or citric acid.

6. The process as claimed in claims 1 to 5, **characterized in that** the diketo ester enolate of the formula (II) is prepared from the corresponding pure, isolated 2,4-diketo ester by addition of an alcoholate in the presence of a solvent.

7. The process as claimed in claims 1 to 6, **characterized in that** portions of the enolate and/or of the N-alkylhydrazinium salt which tend to precipitate are dissolved again or kept in solution by heating and/or addition of further solvent.

8. The process as claimed in claims 1 to 7, **characterized in that** 100 to 2000 ml of solvent are present per mole of reaction batch.

9. The process as claimed in claims 1 to 8, **characterized in that** the molar ratio for the reaction of the diketo ester enolate of the formula (II) with the N-alkylhydrazinium salt of the formula (III) is between 5:1 and 1:5, and the diketo ester enolate of a methyl alkyl ketone of the formula (V) 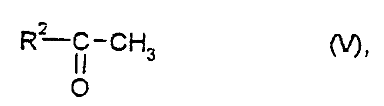 in which
R² has the meaning given in the case of formula (I), has been prepared with an oxalic ester of the formula
R⁴OOC-COOR⁴ (VI)
in which
R⁴ has the meaning given in the case of formula (I)
in the presence of an alcoholate of the formula (IV)
M(OR⁶⁾ ₙ (IV),
in which
M has the meaning in the case of formula (II)
R⁶ represents C₁-C₄-alkyl and
n corresponds to the valency of M, where
the amount of alcohol is at least equimolar and the molar ratio is 0.9 to 0.99 mol of ketone of the formula (V) to 0.9 to 1.1 mol of oxalic acid ester of the formula (VI) to 1 mol of alcoholate of the formula (IV).

10. The process as claimed in claims 1 to 9, **characterized in that** the reaction temperature for the reaction of the reaction partners of the formulae (II) and (III) is between -20 and +100°C and the reaction time is between 0.5 and 12 hours.

## Revendications

1. Procédé pour la préparation d'esters 1-alkyl-pyrazole-5-carboxyliques répondant à la formule (I) 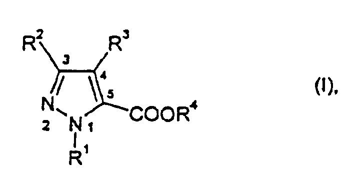 dans laquelle
R¹ et R⁴ représentent, indépendamment l'un de l'autre, respectivement un groupe alkyle en C₁-C₆ à chaîne droite ou ramifiée, un groupe cycloalkyle en C₃-C₇ ou un groupe aralkyle en C₇-C₁₂ portant le cas échéant jusqu'à deux substituants choisis parmi le groupe comprenant des atomes d'halogène et des radicaux alkyle en C₁-C₄ et
R² et R³ représentent, indépendamment l'un de l'autre, respectivement un atome d'hydrogène, un groupe alkyle en C₁-C₆ à chaîne droite ou ramifiée, un groupe cycloalkyle en C₃-C₇ ou un groupe aralkyle en C₇-C₁₂ portant le cas échéant jusqu'à deux substituants choisis parmi le groupe comprenant des atomes d'halogène et des radicaux alkyle en C₁-C₄,
**caractérisé en ce qu'**on ajoute l'énolate d'un ester 2,4-dicétocarboxylique répondant à la formule 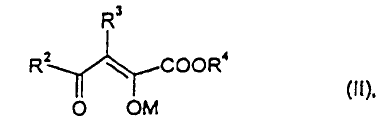 dans laquelle
R², R³ et R⁴ ont la signification indiquée à la formule (I) et
M représente un équivalent d'un atome métallique,
en présence d'un solvant pour obtenir un sel de N-alkylhydrazinium répondant à la formule (III)
R¹-NH₂-NH₂⊕ R⁵COO^{θ} (III),
dans laquelle
R¹ a la signification indiquée à la formule (I) et
R⁵ représente, de manière conjointe avec la fraction COO^{θ}, l'anion d'un acide organique.

2. Procédé selon la revendication 1, **caractérisé en ce que**, en ce qui concerne les énolates d'esters dicétocarboxyliques répondant à la formule (II), il s'agit de ceux dans lesquels les radicaux R² et R³ représentent indépendamment l'un de l'autre, respectivement un atome d'hydrogène, un groupe alkyle en C₁-C₄ à chaîne droite ou ramifiée, un groupe cycloalkyle en C₃-C₆ ou un groupe benzyle le cas échéant substitué, ainsi que ceux dans lesquels le radical R⁴ représente un groupe alkyle en C₁-C₄ à chaîne droite ou ramifiée.

3. Procédé selon les revendications 1 et 2, **caractérisé en ce que** M dans la formule (II) représente le lithium, le sodium, le potassium, le calcium ou le magnésium.

4. Procédé selon les revendications 1 à 3, **caractérisé en ce que**, en ce qui concerne les sels de N-alkylhydrazinium répondant à la formule (III), il s'agit de ceux dans lesquels R¹ représente un groupe alkyle en C₁-C₄ à chaîne droite ou ramifiée ou un groupe benzyle le cas échéant substitué et R⁵ représente un groupe alkyle en C₁-C₆ à chaîne droite ou ramifiée, un groupe cycloalkyle en C₃-C₇, un groupe aralkyle en C₇-C₁₂ ou un groupe aryle en C₆-C₁₀, qui, le cas échéant, peuvent être substitués.

5. Procédé selon les revendications 1 à 4, **caractérisé en ce que**, dans la formule (III), R⁵ représente, de manière conjointe avec la fraction COO^{θ}, un anion d'un acide organique polybasique tel que l'acide oxalique, l'acide malonique, l'acide succinique, l'acide glutarique, l'acide adipique, l'acide maléique, l'acide fumarique, l'acide malique, l'acide tartrique ou l'acide citrique.

6. Procédé selon les revendications 1 à 5, **caractérisé en ce qu'**on prépare les énolates d'esters dicétoniques répondant à la formule (II) à partir des esters 2,4-dicétoniques isolés, purs correspondants par addition d'un alcoolate en présence d'un solvant.

7. Procédé selon les revendications 1 à 6, **caractérisé en ce qu'**on ramène en solution ou on maintient en solution par chauffage et/ou par addition d'une quantité supplémentaire de solvant des fractions des énolates et/ou des sels de N-alkylhydrazinium manifestant une tendance à la précipitation.

8. Procédé selon les revendications 1 à 7, **caractérisé en ce que**, par mole de charge réactionnelle, sont présents de 100 à 2000 ml de solvant.

9. Procédé selon les revendications 1 à 8, **caractérisé en ce que** le rapport molaire pour la mise en réaction des énolates d'esters dicétoniques répondant à la formule (II) avec les sels de N-alkyl-hydrazinium répondant à la formule (III) se situe entre 5 : 1 et 1 : 5 et l'énolate de l'ester dicétonique a été préparé à partir d'une méthylalkylcétone répondant à la formule (V) 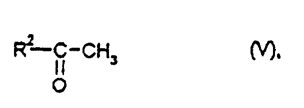 dans laquelle
R² a la signification indiquée à la formule (I)
avec un ester oxalique répondant à la formule
R⁴OOC-COOR⁴ (VI)
dans laquelle
R⁴ a la signification indiquée à la formule (I)
en présence d'un alcoolate répondant à la formule (IV)
M(OR⁶)ₙ (IV)
dans laquelle
M a la signification indiquée à la formule (II)
R⁶ représente un groupe alkyle en C₁-C₄ et
n correspond à la valence de M,
la quantité de l'alcool étant au moins équimolaire et les rapports molaires s'élèvent de 0,9 - 0,99 mole de cétone répondant à la formule (V) à 0,9 - 1,1 mole d'ester oxalique répondant à la formule (VI) à 1 mole d'alcoolate répondant à la formule (IV).

10. Procédé selon les revendications 1 à 9, **caractérisé en ce que** la température réactionnelle pour la mise en réaction des partenaires réactionnels répondant aux formules (II) et (III) se situe entre -20 et +100 °C et les temps de réaction se situent entre 0,5 et 12 heures.
